(19) Europäisches Patentamt
European Patent Office
Office européen des brevets

(11) Veröffentlichungsnummer: **0 200 187**
**B1**

(12) # EUROPÄISCHE PATENTSCHRIFT

(45) Veröffentlichungstag der Patentschrift: **07.06.89**

(51) Int. Cl.$^4$: **C 07 D307/91**

(21) Anmeldenummer: **86105823.8**

(22) Anmeldetag: **28.04.86**

(54) **Verfahren zur Herstellung von 2-Hydroxy-dibenzofuran-3-carbonsäure bzw. deren Alkalisalzen.**

(30) Priorität: **03.05.85 DE 3515873**

(43) Veröffentlichungstag der Anmeldung: **05.11.86 Patentblatt 86/45**

(45) Bekanntmachung des Hinweises auf die Patenterteilung: **07.06.89 Patentblatt 89/23**

(84) Benannte Vertragsstaaten: **CH DE FR IT LI NL**

(56) Entgegenhaltungen:
**US-A- 2 453 105**
**US-A- 2 646 355**
**CHEMICAL ABSTRACTS, Band 55, Nr. 8, 17. April 1961, Columbus, Ohio, USA. P. MADHAVAN NAIR, R. SPRINIVASAN AND K. VENKATARAMAN: "Condensation of 2,4-dinitrochlorobenzene with o-hydroxyarylamides", Zusammenfassung Nr. 7 339i**
**CHEMICAL ABSTRACTS, Band 85, Nr. 8, 23 August 1976, Columbus, Ohio, USA. TERANISHI, KIYOSHI; YOSHIOKA, TOSHIHIRO (MITSUI PETROCHEMICAL INDUSTRIES LTD.): "Recovery of alkali metals from Kolbe.Schmitt reaction products", Seite 194, Spalte 2, Zusammenfassung Nr 49 737q**
**NEUMÜLLER, "Römpps Chemie Lexikon", 8. Auflage, Band I, "Butanol", 1979. FRANCKH'SCHE VERLAGSHANDLUNG; Stuttgart, Seite 540**
**HOUBEN-WEYL "Methoden der organischen Chemie"; 4. Auflage, Band VIII, 1952, GEORG THIEME VERLAG, Stuttgart, Seiten 372-373**
**CHEMICAL ABSTRACTS, Band 72, Nr. 10, 9 März 1970, Columbus, Ohio, USA. BHAGWANTH, M.R.R.; RAO, A.V. RAMA; VENKATARAMAN, K. "Applications of NMR and mass spectroscopy to some problems**

(73) Patentinhaber: **HOECHST AKTIENGESELLSCHAFT**
**Postfach 80 03 20**
**D-6230 Frankfurt am Main 80 (DE)**

(72) Erfinder: **Neeb, Rudolf, Dr.**
**Am Klingenrain 23**
**D-6050 Offenbach am Main (DE)**
Erfinder: **Tronich, Wolfgang, Dr.**
**Adolf-Guckes-Weg 5**
**D-6239 Eppstein/Taunus (DE)**

**EP 0 200 187 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

concerning synthetic dyes. V. Kolbe-Schmitt reaction products from 2-hydroxycarbazole and the structure of Naphthol AS-LB" Seite 79, Spalte 1, Zusammenfassung Nr. 44 997w
CHEMICAL ABSTRACTS, Band 80, Nr. 17, 29 April 1974, Columbus, Ohio, USA. KAMEL, M.; ALLAM, M.A.; EL-ALFI, I. "Synthesis of some dinaphthoxanthones, benzonaphthoxanthones and benzofuroxanthones", Seite 388, Spalte 1, Zusammenfassung Nr 95 662f

## Beschreibung

Die Erfindung liegt auf dem technischen Gebiet der Synthese von Zwischenprodukten, die bevorzugt in der Herstellung von Farbstoffen verwendet werden.

2-Hydroxy-dibenzofuran-3-carbonsäure der Formel

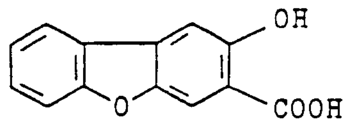

ist insbesondere in Form ihrer Arylamide ein Vorprodukt für die Herstellung von wasserunlöslichen Azofarbstoffen (s. bspw. deutsches Reichspatent Nr. 607 381, abgedruckt in Friedländer, Fortschritte der Teerfarbenfabrikation, 21, 275, und Colour Index C. I.-Nr. 37605). Sie wird durch Carboxylierung der Alkalisalze von 2-Hydroxy-dibenzofuran gemäß der Kolbe-Schmitt-Reaktion hergestellt (s. deutsche Reichspatentschrift Nr. 593 506, abgedruckt in Friedländer 20, 487). Eine detaillierte Arbeitsweise dieser Carboxylierungsreaktion ist in BIOS Final Report Nr. 1149, Seite 93, beschrieben. Dort wird 2-Hydroxy-dibenzofuran (in BIOS als 3-Hydroxy-diphenylenoxid bezeichnet) in Form dessen trockenen Natriumsalzes unter einem Kohlendioxid-Druck von etwa 4,5 bar während etwa 20 Stunden bei einer Temperatur von 260 bis 265 °C carboxyliert, wobei der Reaktionsansatz nach etwa 10 Stunden unterbrochen und der Umsetzungsgrad geprüft werden muß. Das als Ausgangsverbindung dienende trockene Natriumsalz des 2-Hydroxy-dibenzofurans muß zuvor umständlich durch Erhitzen von 2-Hydroxydibenzofuran mit wäßriger Natronlauge bei 145 bis 150 °C während 6 Stunden und durch anschließendes Eindampfen der gebildeten Paste unter reduziertem Druck bei einer Temperatur bis 225 °C während 8 Stunden hergestellt werden. Die Aufarbeitung des Carboxylierungsgemisches nach beendeter Carboxylierungsreaktion erfolgt unter Aufteilung dieses Ansatzes in vier getrennte Aufarbeitungsansätze. Hierbei wird jeder Teil zunächst in der 66-fachen Gewichtsmenge Wasser, dem etwas Bisulfitlauge zugesetzt ist, gelöst. Die Lösung wird mit Schwefelsäure zunächst schwach sauer, anschließend mit Natriumcarbonat genau neutral gestellt; nach deren Klärfiltration wird hieraus die 2-Hydroxy-dibenzofuran-3-carbonsäure mit geringem Schwefelsäureüberschuß ausgefällt und in einer Ausbeute von 84 % d. Th. erhalten. Sie ist noch mit freiem 2-Hydroxydibenzofuran verunreinigt. Liegt deren Gehalt an freiem 2-Hydroxy-dibenzofuran bei 2 bis 4 Gew.-% und höher, so muß es aus Qualitätsgründen von der 2-Hydroxydibenzofuran-3-carbonsäure durch ein aufwendiges Sublimationsverfahren entfernt werden.

Es liegt auf der Hand, daß dieses bekannte Verfahren mehrere schwerwiegende Mängel aufweist, die nach den heutigen Anforderungen der wirtschaftlichen Herstellung dieses Produktes entgegenstehen. So sind insbesondere die lange Entwässerungsphase zur Herstellung des trockenen Natriumsalzes des 2-Hydroxy-dibenzofurans, die lange Carboxylierungsphase und insbesondere der große Volumenbedarf bei der Auslösung der Carboxylierungsmasse, der deren Aufteilung bei der Aufarbeitung erzwingt, nicht mehr zu vertreten. Ein weiterer Nachteil des bekannten Verfahrens besteht schließlich darin, daß das für die Weiterverarbeitung durchaus erwünschte Alkalisalz der 2-Hydroxy-dibenzofuran-3-carbonsäure nur mit technisch nicht vertretbarem Aufwand aus der hochverdünnten wäßrigen Lösung in Substanz isoliert werden kann.

Es stellte sich somit die Aufgabe, ein Verfahren zu finden, das diese Nachteile nicht mehr besitzt. Die Aufgabe wurde mit der vorliegenden Erfindung gelöst.

Die Erfindung betrifft somit ein Verfahren zur Hestellung von 2-Hydroxy-dibenzofuran-3-carbonsäure, insbesondere in Form der Alkalisalze, durch Umsetzung eines Alkalisalzes des 2-Hydroxy-dibenzofuran mit Kohlendioxid bei einer Temperatur oberhalb von 120 °C, das dadurch gekennzeichnet ist, daß man die Umsetzung in einem aliphatischen Alkohol mit 4 bis 8 C-Atomen durchführt. Die Umsetzung kann hierbei ohne Kohlendioxid-Druck oder unter Kohlendioxid-Druck erfolgen. Bevorzugt erfolgt die Umsetzung unter geringem Kohlendioxid-Druck.

Auf diese Weise erhält man das Alkalisalz der 2-Hydroxydibenzofuran-3-carbonsäure in hoher Reinheit und guter Ausbeute beispielsweise direkt aus deren alkoholischen Suspension.

Erfindungsgemäß verwendete Löse- bzw. Verdünnungsmittel sind insbesondere geradkettige oder verzweigte Alkanole von 4 bis 8 C-Atomen mit einem Siedepunkt von über 100 °C. Besonders bevorzugt eignen sich Hexanole und Octanole, insbesondere das 2-Ethyl-hexanol. Die erfindungsgemäß verwendeten aliphatischen Alkohole können allein oder im Gemisch eingesetzt werden.

Die als Ausgangsverbindungen dienenden Alkalisalze des 2-Hydroxy-dibenzofurans sind im wesentlichen das Natrium- und Kaliumsalz, bevorzugt das Natriumsalz. Sie können nach bekannten Verfahren in einem getrennten Schritt zuvor in wasserfreier Form hergestellt und sodann in das erfindungsgemäß verwendete Löse- bzw. Verdünnungsmittel eingebracht werden. Es ist jedoch vorteilhaft, die Herstellung des Alkalisalzes in das erfindungsgemäße Verfahren in der Weise zu integrieren, daß man das 2-Hydroxy-dibenzofuran zusammen mit einem Alkalihydroxid, beispielsweise in Form einer wäßrigen Alkalilauge, gemeinsam in das erfindungsgemäß verwendete Lösemittel einbringt und hieraus das Wasser durch Abdestillieren entfernt. Hierbei werden gegebenenfalls auch Anteile des Lösemittels abdestilliert, das jedoch leicht wieder ergänzt werden kann.

Eine andere Verfahrensweise der Herstellung des Alkalisalzes des 2-Hydroxy-dibenzofurans ist dadurch gekennzeichnet, daß man das 2-Hydroxy-dibenzofuran in einem längerkettigen aliphatischen Alkohol, wie einem Hexanol oder Octanol, so beispielsweise in 2-Ethyl-hexanol, mit der Lösung der erforderlichen Menge eines Alkalialkoholats eines bevorzugt niederen aliphatischen Alkohols in einem bevorzugt niederen aliphatischen Alkohol, wie beispielsweise eine Lösung von Natriummethylat in Methanol, versetzt und dieses Gemisch unter Abdestillieren des niederen Alkohols und gegebenenfalls eines Teils des längerkettigen Alkohols erhitzt.

Das Alkalisalz des 2-Hydroxy-dibenzofurans kann in die Carboxylierungsreaktion mit einem Überschuß an Alkali eingesetzt werden. Im allgemeinen liegt das molare Verhältnis von Alkalihydroxid zu 2-Hydroxy-dibenzofuran als Komponenten des Alkalisalzes des 2-Hydroxy-dibenzofurans bei (0,9-1,5) : 1, bevorzugt bei (0,98-1,3) : 1, insbesondere jedoch in einem molaren Verhältnis von (1,0-1,1) : 1. Das 2-Hydroxy-dibenzofuran-Alkalisalz wird in den erfindungsgemäßen Reaktionsansatz in der Regel in einer Konzentration zwischen 5 und 50 Gew.-%, bevorzugt zwischen 10 und 25 Gew.-%, bezogen auf das erfindungsgemäß verwendete Löse- bzw. Verdünnungsmittel, eingesetzt.

Die Carboxylierungstemperatur liegt im allgemeinen zwischen 150 und 240 °C. Bevorzugt arbeitet man bei einer Temperatur zwischen 180 und 230 °C, insbesondere zwischen 215 und 225 °C. Sofern man unter Kohlendioxid-Druck arbeitet, kann dieser in weiten Bereichen variieren. Im allgemeinen liegt er zwischen 0,5 und 50 bar, bevorzugt zwischen 2 und 10 bar, insbesondere zwischen 3 und 6 bar. Arbeitet man ohne Kohlendioxid-Druck, so wird diese Verfahrensvariante insbesondere bei Verwendung eines hochsiedenden Alkanols ausgeübt. Bevorzugt arbeitet man jedoch unter einem mehr oder weniger geringen Kohlendioxid-Überdruck. Das für die Umsetzung erforderliche Kohlendioxid kann in den Reaktionsansatz beispielsweise durch Einleiten des Kohlendioxidgases in die alkoholische Suspension des Alkalisalzes des 2-Hydroxydibenzofurans oder durch Überleiten des Kohlendioxidgases über diese alkoholische Suspension eingebracht werden.

Der Carboxylierungsansatz kann nach beendeter Carboxylierungsreaktion in einer dem Fachmann an und für sich geläufigen Weise aufgearbeitet werden. So kann in einfacher Weise das gebildete 2-Hydroxy-dibenzofuran-3-carbonsäure-Alkalisalz aus der Suspension des verfahrensgemäß verwendeten Löse- bzw. Verdünnungsmittels bei Raumtemperatur oder bei erhöhter Temperatur, beispielsweise durch Abfiltrieren oder Zentrifugieren, isoliert werden. Man kann auch so verfahren, daß man das verfahrensgemäß verwendete Lösemittel zunächst weitgehend oder vollständig abdestilliert, das zurückbleibende Alkalisalz der 2-Hydroxy-dibenzofuran-3-carbonsäure in Wasser suspendiert und es sodann in üblicher Weise aus der wäßrig-organischen bzw. wäßrigen Suspension isoliert. Eine weitere Möglichkeit der Isolierung des Alkalisalzes der 2-Hydroxydibenzofuran-3-carbonsäure ist im Falle, daß man bei der Carboxylierungsreaktion ein genügend Wasserdampf-flüchtiges Löse- bzw. Verdünnungsmittel verwendet, dieses flüchtige Lösemittel mit Wasserdampf abzudestilliegen und aus der verbleibenden wäßrigen Suspension das gewünschte Alkalisalz zu isolieren.

Zur Gewinnung der 2-Hydroxy-dibenzofuran-3-carbonsäure selbst kann man die Suspension des Alkalisalzes in üblicher Weise, beispielsweise mit Mineralsäuren, wie Salz- oder Schwefelsäure, sauer stellen und aus der sauren Suspension die freigewordene 2-Hydroxy-dibenzofuran-3-carbonsäure durch Filtration oder Zentrifugieren isolieren.

Das erfindungsgemäße Verfahren der Carboxylierungsreaktion, einschließlich der eventuell integrierten vorangehenden Salzbildung des 2-Hydroxy-dibenzofurans und der Entwässerung des Reaktionsansatzes läßt sich in üblichen technischen Apparaturen ausführen, so in mit einem Rührmechanismus versehenen verschließbaren Kessel (Autoklaven), der mit einer einfachen Destillationsapparatur und -vorlage versehen sein kann und mit dem gegebenenfalls auch unter höheren Drücken gearbeitet werden kann.

Eine bevorzugte Ausführungsform des erfindungsgemäßen Verfahrens kann beispielsweise wie folgt beschrieben werden: Zur Überführung des 2-Hydroxy-dibenzofurans in das als Ausgangsverbindung dienende Alkalisalz erhitzt man zunächst eine Lösung oder Suspension des 2-Hydroxy-dibenzofurans in einem Alkanol von 4 bis 8 C-Atomen mit einem Siedepunkt von über 100 °C mit der äquivalenten Menge einer wäßrigen Alkalilauge, wie einer wäßrigen Lösung von Natriumhydroxid oder Kaliumhydroxid, auf eine Temperatur von oberhalb 100 °C bei steigender Temperatur solange, bis kein Wasser oder kein Alkohol/Wasser-Gemisch mehr überdestilliert. Eventuell abdestillierte Alkoholmengen werden, soweit erforderlich, anschließend ersetzt. Diese Alkalisalzbildung und Entwässerung geschieht in der Regel unter einer Stickstoffatmosphäre. Als Löse- bzw. Verdünnungsmittel wird bevorzugt ein Octanol, insbesondere 2-Ethylhexanol, verwendet. Nach dieser Salzbildung erfolgt die Carboxylierungsreaktion unter Einleiten und Aufdrücken von Kohlendioxidgas in geschlossener Apparatur auf die Suspension des Alkalisalzes des 2-Hydroxy-dibenzofurans in dem erfindungsgemäß verwendeten Löse- oder Verdünnungsmittel. Bevorzugt wird ein $CO_2$-Druck von 3 bis 6 bar gewählt. Hierbei wird die Temperatur des Reaktionsansatzes auf 180 bis 240, bevorzugt auf 215 bis 225 °C erhöht und die Carboxylierungsreaktion innerhalb dieses Temperatur- und $CO_2$-Druckbereiches über einige Stunden weiter- und zu Ende führt. In der Regel reicht eine Reaktionszeit von 3 bis 6 Stunden aus. Danach kühlt man das Reaktionsgemisch auf etwa 80 bis 100 °C ab und isoliert das gebildete Alkalisalz der 2-Hydroxy-dibenzofuran-3-carbonsäure, beispielsweise durch Filtration.

Das Filtrat selbst kann in Nachfolgeansätze mehrmals wiederverwendet werden.

Die nachfolgenden Beispiele dienen zur näheren Erläuterung des Erfindungsgegenstandes. Teile sind Gewichtsteile, und die Prozentangaben beziehen sich auf Gewichtsprozente, sofern nicht anders vermerkt.

Beispiel 1

Von einem Gemisch, bestehend aus 184 Teilen 2-Hydroxydibenzofuran 1300 Teilen 2-Ethyl-hexanol und 180 Teilen einer methanolischen Lösung mit einem Gehalt von 30 % Natriummethylat, wird in einem Rührautoklaven das Methanol bei Normaldruck abdestilliert; zu Ende der Destillation wird die Innentemperatur bis zum Siedepunkt des 2-Ethylhexanols gesteigert, wobei etwa 200 Teile 2-Ethylhexanol abdestilliert werden. Sodann erhitzt man die gebildete Suspension des Natriumsalzes des 2-Hydroxy-dibenzofurans in 2-Ethyl-hexanol im geschlossenen Autoklaven auf 220 °C und drückt mit 4 bar Kohlendioxid auf. Die Carboxylierungsreaktion wird unter Aufrechterhaltung des $CO_2$-Druckes von 4 bar und der Reaktionstemperatur von 220 °C noch 4 Stunden weitergerührt. Anschließend wird der Ansatz auf etwa 100 °C abgekühlt, das Produkt abgesaugt, der Filterrückstand mit Ethanol gewaschen und bei 60 °C getrocknet.

Es werden 198 Teile 2-Hydroxy-dibenzofuran-3-carbonsäure-Natriumsalz mit einem Reingehalt von 94 % gleich einer Ausbeute von 74,5 % d. Th. erhalten (das Produkt enthält etwa 6 % Natriumcarbonat).

Das aus der Carboxylierungsreaktion nach Abtrennung des Syntheseproduktes erhaltene Filtrat wird einschließlich der ethanolischen Waschflüssigkeit unter reduziertem Druck zur Trockene eingedampft. Der Rückstand wird in Wasser aufgenommen, die Lösung mit etwas Aktivkohle oder Kieselgur geklärt und sodann mit Mineralsäure, wie Salzsäure, auf einen pH-Wert zwischen 7,0 und 6,8 eingestellt. Hierbei fallen etwa 32,5 Teile nicht umgesetztes 2-Hydroxy-dibenzofuran aus, das in einen Nachfolgeansatz wieder eingesetzt werden kann.

Die Ausbeute an 2-Hydroxy-dibenzofuran-3-carbonsäure, bezogen auf umgesetztes 2-Hydroxy-dibenzofuran, beträgt somit 90,5 %.

Beispiel 2

In einem Rührautoklaven wird aus einem Gemisch von 184 Teilen 2-Hydroxy-dibenzofuran 129 Teilen einer 48 %igen wäßrigen Kaliumhydroxidlösung und 1300 Teilen 2-Ethyl-hexanol das Wasser vollständig unter Überleiten von Stickstoff und stetiges Erhitzen oberhalb 110 °C abdestilliert. Nach Erreichen eines Siedepunktes von etwa 184 °C werden noch 200 Teile 2-Ethyl-hexanol abdestilliert und sodann der Ansatz im geschlossenen Autoklaven auf 220 °C unter Einleiten von Kohlendioxid mit einem Druck von 3 bar erhitzt. Die Carboxylierungsreaktion wird noch 4 Stunden unter Aufrechterhaltung dieses $CO_2$-Druckes und der Reaktionstemperatur weitergeführt. Nach Abkühlen des Carboxylierungsansatzes auf etwa 100 °C wird das Reaktionsprodukt abgesaugt, der Filterrückstand mit Ethanol gewaschen und bei etwa 60 °C getrocknet.

Man erhält 209 Teile des 2-Hydroxy-dibenzofuran-3-carbonsäure-Kaliumsalzes mit einem Reingehalt von 92 % entsprechend einer Ausbeute an dem reinen Produkt von 72,1 % d. Th. (das Produkt enthält etwa 8 % Kaliumcarbonat).

Das im Filtrat verbliebene nicht umgesetzte 2-Hydroxy-dibenzofuran kann gemäß den Angaben des Beispieles 1 zurückgewonnen werden.

Die Ausbeute an 2-Hydroxy-dibenzofuran-3-carbonsäure, bezogen auf umgesetztes 2-Hydroxy-dibenzofuran, beträgt somit 87,6 %.

Beispiel 3

Man verfährt zur Herstellung des Natriumsalzes der 2-Hydroxy-dibenzofuran-3-carbonsäure gemäß der Verfahrensweise des Beispieles 1, jedoch mit dem Unterschied, daß man die Umsetzung bei einem Kohlendioxid-Druck von 6 bar durchführt. Man erhält das gewünschte Endprodukt in der im Beispiel 1 angegebenen Ausbeute und Reinheit.

Beispiel 4

Zur Herstellung des Natriumsalzes der 2-Hydroxy-dibenzofuran-3-carbonsäure verfährt man gemäß der Verfahrensweise des Beispieles 1, jedoch mit dem Unterschied, daß man anstelle der angegebenen Menge von 2-Ethyl-hexanol das nicht aufgearbeitete Filtrat ohne die ethanolische Waschflüssigkeit aus vorhergehenden Ansätzen in gleicher Menge einsetzt, wobei man zusätzlich wegen des Gehaltes des Filtrats an 2-Hydroxy-dibenzofuran bzw. an dessen Natriumsalz lediglich 151,5 Teile 2-Hydroxy-dibenzofuran und die äquivalente Menge von 148 Teilen einer methanolischen Lösung mit 30% Natriummethylat hinzufügt.

Man erhält 197 Teile des gewünschten Natriumsalzes der 2-Hydroxy-dibenzofuran-3-carbonsäure mit einem Reingehalt von 94 %.

Auch hier kann das Filtrat wiederum einem Nachfolgeansatz zugeführt werden.

## Patentansprüche

1. Verfahren zur Herstellung von 2-Hydroxy-dibenzofuran-3-carbonsäure, insbesondere in Form der Alkalisalze, durch Umsetzung eines Alkalisalzes des 2-Hydroxydibenzofurans mit Kohlendioxid bei einer Temperatur oberhalb von 120 °C, dadurch gekennzeichnet, daß man die Umsetzung in einem aliphatischen Alkohol von 4 bis 8 C-Atomen durchführt.

2. Verfahren nach Anspruch 1, dadurch gekennzeichnet, daß man die Umsetzung unter Kohlendioxid-Druck durchführt.

3. Verfahren nach Anspruch 1 oder 2, dadurch gekennzeichnet, daß man ein Octanol als Löse- bzw. Verdünnungsmittel verwendet.

4. Verfahren nach Anspruch 1 oder 2, dadurch gekennzeichnet, daß man 2-Ethyl-hexanol als Löse- bzw. Verdünnungsmittel verwendet.

5. Verfahren nach einem der Ansprüche 1 bis 4, dadurch gekennzeichnet, daß man die Carboxylierungsreaktion bei einer Temperatur zwischen 180 und 230 °C durchführt.

6. Verfahren nach einem der Ansprüche 1 bis 4, dadurch gekennzeichnet, daß man die Carboxylierungsreaktion bei einer Temperatur zwischen 215 und 225 °C durchführt.

7. Verfahren nach einem der Ansprüche 1 bis 6, dadurch gekennzeichnet, daß man das Löse- bzw. Verdünnungsmittel aus dem Carboxylierungsansatz ohne Regeneration in einen Nachfolgeansatz einsetzt.

8. Verfahren nach einem der Ansprüche 2 bis 7, dadurch gekennzeichnet, daß die Carboxylierungsreaktion bei einem Kohlendioxid-Druck zwischen 2 und 10 bar durchgeführt wird.

9. Verfahren nach einem der Ansprüche 1 bis 8, dadurch gekennzeichnet, daß man das Alkalisalz des 2-Hydroxydibenzofurans zünächst durch Erhitzen von 2-Hydroxybenzofuran und einem wäßrigen Alkali in einem Alkanol von 4 bis 8 C-Atomen unter Abdestillation von Wasser erzeugt und anschließend, ohne das Alkalisalz abzutrennen, die Suspension des Alkalisalzes des 2-Hydroxybenzofurans in dem Alkanol der Carboxylierungsreaktion gemäß Anspruch 1 unterwirft.

10. Verfahren nach einem der Ansprüche 1 bis 9, dadurch gekennzeichnet, daß man zur Überführung des gebildeten Alkalisalzes der 2-Hydroxy-benzofuran-3-carbonsäure in die 2-Hydroxy-benzofuran-3-carbonsäure die nach der Carboxylierung erhaltene Suspension dieses Alkalisalzes mit einer Mineralsäure ansäuert und die unlösliche Carbonsäure daraus isoliert.

## Claims

1. A process for preparing 2-hydroxydibenzofuran-3-carboxylic acid, in particular in the form of an alkali metal salt, by reacting an alkali metal salt of 2-hydroxydibenzofuran with carbon dioxide at a temperature above 120 °C, which comprises carrying out the reaction in an aliphatic alcohol of 4 to 8 carbon atoms.

2. The process as claimed in claim 1, wherein the reaction is carried out under carbon dioxide pressure.

3. The process as claimed in claim 1 or 2, wherein an octanol is used as a solvent or diluent.

4. The process as claimed in claim 1 or 2, wherein 2-ethylhexanol is used as the solvent or diluent.

5. The process as claimed in any one of claims 1 to 4, wherein the carboxylation reaction is carried out at a temperature between 180 and 230 °C.

6. The process as claimed in any one of claims 1 to 4, wherein the carboxylation reaction is carried out at a temperature between 215 and 225 °C.

7. The process as claimed in any one of claims 1 to 6, wherein the solvent or diluent from the carboxylation batch is used without regeneration in a subsequent batch.

8. The process as claimed in any one of claims 2 to 7, wherein the carboxylation reaction is carried out under a carbon dioxide pressure between 2 and 10 bar.

9. The process as claimed in any one of claims 1 to 8, wherein, first, the alkali metal salt of 2-hydroxydibenzofuran is produced by heating 2-hydroxydibenzofuran and an aqueous alkali in an alkanol of 4 to 8 carbon atoms while distilling off water and, subsequently, without removal of the alkali metal salt formed, the suspension of the alkali metal salt of 2-hydroxybenzofuran in that alkanol is subjected to the carboxylation reaction according to claim 1.

10. The process as claimed in any one of claims 1 to 9, wherein, to convert the alkali metal salt of 2-hydroxy-benzofuran-3-carboxylic acid formed into the 2-hydroxy-benzofuran-3-carboxylic acid, the suspension of this alkali metal salt obtained from the carboxylation is acidified with a mineral acid and the insoluble carboxylic acid is isolated therefrom.

## Revendications

1. Procédé de fabrication de l'acide 2-hydroxy-dibenzofuranne-3-carboxylique, et en particulier de

sels de métaux alcalins de cet acide, par réaction d'un sel alcalin du 2-hydroxy-dibenzofuranne avec le dioxyde de carbone à une température supérieure à 120 °C, procédé caractérisé en ce que l'on effectue cette réaction dans un alcool aliphatique ayant de 4 à 8 atomes de carbone.

2. Procédé selon la revendication 1 caractérisé en ce que l'on effectue la réaction sous une pression du dioxyde de carbone.

3. Procédé selon la revendication 1 ou 2, caractérisé en ce que l'on utilise un octanol comme solvant ou diluant.

4. Procédé selon la revendication 1 ou 2, caractérisé en ce que l'on utilise le 2-éthyl-hexanol comme solvant ou diluant.

5. Procédé selon l'une quelconque des revendications 1 à 4, caractérisé en ce que l'on effectue la réaction de carboxylation à une température comprise entre 180 et 230 °C.

6. Procédé selon l'une quelconque des revendications 1 à 4, caractérisé en ce que l'on effectue la réaction de carboxylation à une température comprise entre 215 et 225 °C.

7. Procédé selon l'une quelconque des revendications 1 à 6, caractérisé en ce que l'on réemploie dans une opération suivante le solvant ou diluant du mélange de carboxylation, sans le régénérer.

8. Procédé selon l'une quelconque des revendications 2 à 7, caractérisé en ce que l'on effectue la carboxylation sous une pression du dioxyde de carbone comprise entre 2 et 10 bars.

9. Procédé selon l'une quelconque des revendications 1 à 8, caractérisé en ce que l'on commence par former le sel alcalin du 2-hydroxy-dibenzofuranne en chauffant celui-ci avec un alcali aqueux dans un alcanol en $C_4$ à $C_8$ tout en éliminant l'eau par distillation, puis, sans séparer le sel formé, on soumet sa suspension dans l'alcanol à la réaction de carboxylation conformément à la revendication 1.

10. Procédé selon l'une quelconque des revendications 1 à 9, caractérisé en ce que pour transformer en acide libre le sel alcalin formé de l'acide 2-hydroxy-dibenzofuranne-3-carboxylique, on acidifie avec un acide minéral la suspension du sel alcalin résultant de la carboxylation et on en isole l'acide carboxylique insoluble.